# EUROPEAN PATENT APPLICATION

(11) **EP 4 643 850 A1**
(43) Date of publication of application: **05.11.2025**
(21) Application number: 23909780.1
(22) Date of filing: 21.11.2023
(51) Int. Cl.: A61K 9/16, A61K 9/127, A61K 47/34, A61K 47/28, A61K 47/24, A61P 31/12

(54) **NUCLEIC ACID DELIVERY CARRIER, PREPARATION METHOD THEREFOR, AND USE THEREOF**

(30) Priority: 29.12.2022 CN 202211701414
(71) Applicant: Cansino (Shanghai) Biological Research Co., Ltd., Shanghai 201203 (CN); Cansino (Shanghai) Biotechnologies Co.,Ltd, Shanghai 201208 (CN)
(72) Inventor: WANG, Haomeng, Shanghai 201203 (CN); LI, Jin, Shanghai 201203 (CN); WANG, Zhenghua, Shanghai 201203 (CN); YAN, Zhihong, Shanghai 201203 (CN); CHAO, Shoubai, Shanghai 201203 (CN); YU, Xuefeng, Shanghai 201203 (CN); LIU, Jian, Shanghai 201203 (CN); QIU, Dongxu, Shanghai 201203 (CN); ZHU, Tao, Shanghai 201203 (CN)
(74) Representative: Finnegan Europe LLP
(86) International application number: PCT/CN2023/132937
(87) International publication number: WO 2024/139848

(57) **Abstract**

A lipid nanoparticle composition, a preparation method therefor, and use thereof in nucleic acid delivery. An mRNA drug or vaccine can be prepared on the basis of the lipid nanoparticle composition. The starting materials of the lipid nanoparticle composition comprise an ionizable cationic lipid, an auxiliary phospholipid, a sterol compound, a lipid polyethylene glycol conjugate, and a buffer.

## Description

### TECHNICAL FIELD

The present disclosure relates to the technical field of biopharmaceutical formulations, in particular to an optimized lipid nanoparticle composition formed by a combination of a cationic lipid compound, a helper phospholipid, sterol, and a PEG-lipid and a buffer system, which is used for delivery of nucleic acid drugs.

### BACKGROUND

Nucleic acid drugs have a phosphodiester structure with high electronegativity, which makes it difficult for the nucleic acid drugs to enter cells and exert their effects. Meanwhile, due to the property of easy degradation, the nucleic acid drugs need to be encapsulated by a delivery carrier for intracellular delivery. Therefore, the exploitation of a delivery carrier with safety and efficacy is a critical bottleneck part in the development of nucleic acid drugs.

Delivery carriers currently used for nucleic acid drugs include viral carriers and non-viral carriers, and the non-viral carriers include lipid nanoparticles, polymer nanoparticles, and the like. The lipid nanoparticles are currently the most popular delivery carriers in use, including two mRNA vaccines that have been marketed. A lipid nanoparticle delivery system is generally composed of four components, i.e., an ionizable lipid compound (or cationic lipid compound), a helper lipid compound, cholesterol, and lipid-PEG. Two marketed COVID-19 mRNA vaccines both use LNP as the delivery carrier, but their stability is both relatively poor. For example, mRNA-1273 from Modema has a storage life of 9 months at -25 °C to -15 °C and a storage life of 30 days at 2 °C to 8 °C; while BNT162b2 from Pfizer-BioNTech has a storage life of 9 months at -90 °C to -60 °C and a storage life of 1 month at 2 °C to 8 °C. Therefore, the application scope of such mRNA-LNPs is limited to some extent.

The current formulation formula for lipid nanoparticles requires some optimization in terms of stability and safety to meet the needs of long-distance transportation and large-scale vaccination.

### SUMMARY

The present disclosure aims to provide a lipid nanoparticle composition for nucleic acid drug delivery. Such lipid nanoparticles have better stability and safety than existing lipid nanoparticle carriers.

The present disclosure further provides a formulation auxiliary material comprising the lipid nanoparticle complex described above.

The present disclosure further provides a nucleic acid-lipid nanoparticle composition comprising the carrier described above, and proportions.

The present disclosure further provides a pharmaceutical formulation comprising the lipid carrier described above or the nucleic acid-lipid nanoparticle composition described above. The technical solutions adopted by the present disclosure are as follows.

The present disclosure provides a lipid nanoparticle composition for nucleic acid drug delivery, comprising the following starting materials:
(a) an ionizable cationic lipid, wherein preferably, the ionizable cationic lipid accounts for 30%-80% of total lipid present in the lipid nanoparticle on the basis of molar percentage;
(b) a helper phospholipid, wherein preferably, the helper phospholipid is DSPC, and more preferably, the helper phospholipid accounts for 1%-20% of total lipid present in the lipid nanoparticle on the basis of molar percentage;
(c) a sterol compound, wherein preferably, the sterol compound is cholesterol, and more preferably, the sterol compound accounts for 10%-60% of total lipid present in the lipid nanoparticle on the basis of molar percentage;
(d) a lipid-polyethylene glycol conjugate, wherein preferably, the lipid-polyethylene glycol conjugate is one or more of DMG-PEG2000 and ALC-0159, and more preferably, the lipid-polyethylene glycol conjugate accounts for 0.1%-15% of total lipid present in the lipid nanoparticle on the basis of molar percentage;
(e) a buffer, wherein preferably, the buffer is selected from one or more of a tris buffer, an acetate buffer, a citrate buffer, and a phosphate buffer.

Specifically, the ionizable cationic lipid is one or more of ALC-0315 and SM-102; more specifically, the buffer has a pH of 5-9, preferably a pH of 6-8.5.

Specifically, the ALC-0315, the helper phospholipid DSPC, the cholesterol, and the DMG-PEG2000 are in a molar ratio of (45%-60%):(5%-15%):(30%-45%):(0.5%-5%).

Specifically, the buffer is at a concentration of 1-100 mM, preferably a concentration of 5-50 mM.

Specifically, the total lipid comprises an ionizable cationic lipid, a helper phospholipid, a sterol compound, a lipid-polyethylene glycol conjugate, and the like.

Specifically, the composition further comprises an additional auxiliary material, wherein the additional auxiliary material comprises one of sodium acetate, tromethamine, potassium dihydrogen phosphate, sodium chloride, disodium hydrogen phosphate, and sucrose or combinations thereof.

Specifically, the composition further comprises a nucleic acid drug.

Specifically, the nucleic acid drug includes ssDNA, dsDNA, mRNA, lncRNA, siRNA, saRNA, shRNA, ASO, a plasmid, circRNA, circDNA, miRNA, CRISPR-Cas, ployI:C, SamRNA, or 5'-pppRNA; preferably, the nucleic acid drug is mRNA.

Specifically, the composition is a liquid formulation or a lyophilized powder formulation; preferably, the composition is a formulation for oral administration, intramuscular injection, intravenous injection, or inhalation.

The present disclosure provides a preparation method for the lipid nanoparticle composition, wherein the composition is prepared by dissolving the ionizable cationic lipid, the helper phospholipid, the sterol compound, and the PEG-lipid conjugate in a solvent, mixing the solution with a nucleic acid drug, diluting the mixture with the buffer, and concentrating the dilution.

Specifically, the mass ratio (w/w) of the total lipid in the lipid nanoparticle (LNP) to mRNA is (10-30):1.

Specifically, the lipid nanoparticle has an average particle size of 50-200 nm; or the lipid nanoparticle has a net neutral charge at neutral pH; or the lipid nanoparticle has a polydispersity value of less than 0.4.

Specifically, the lipid nanoparticle has an N/P ratio of 1-10 when encapsulating mRNA.

The present disclosure provides use of the lipid nanoparticle composition in the manufacture of a prophylactic or therapeutic medicament, wherein preferably, the medicament is a vaccine, and more preferably, the vaccine is a vaccine for preventing a cancer, a viral infection, a bacterial infection, or a fungal infection; more preferably, the virus is selected from norovirus, Ebola virus, coronavirus, cytomegalovirus, dengue virus, Zika virus, coxsackievirus, enterovirus, hepatitis virus, herpes simplex virus, human papilloma virus, influenza virus, Marburg virus, measles virus, poliovirus, rabies virus, rotavirus, and measles virus.

Compared with the prior art, the present disclosure has beneficial effects in that the present disclosure provides a lipid nanoparticle complex and a formulation auxiliary material thereof, which can be used for nucleic acid drug delivery. By studying and controlling the encapsulation efficiency, particle size, PDI, and potential of lipid nanoparticles, as well as adjusting and optimizing the cationic lipid content in the lipid nanoparticle complex, the obtained lipid nanoparticle complex formulations have better immunogenicity. Such lipid nanoparticle complex formulations have better stability and safety for mRNA drugs than existing lipid nanoparticle carriers.

### BRIEF DESCRIPTION OF THE DRAWINGS

FIG. 1 is a graph showing data for average particle size of LNP formulations.
FIG. 2 is a graph showing data for encapsulation efficiency of LNP formulations.
FIG. 3 is a graph showing data for mRNA integrity of LNP formulations.
FIG. 4 is a graph showing data for PDI of LNP formulations.
FIG. 5 is an example image showing scoring of an inflammatory response (0 points).
FIG. 6 is an example image showing scoring of an inflammatory response (1 point).
FIG. 7 is an example image showing scoring of an inflammatory response (2 points).
FIG. 8 is an example image showing scoring of an inflammatory response (3 points).
FIG. 9 is a graph showing data for scoring of an inflammatory response.
FIG. 10 is a graph showing data for assay values of cytokines.
FIG. 11 is a graph showing data for assay values of antibody titer GMT (95% CI).
FIG. 12 is a graph showing the comparison of the incidences of clinical adverse reactions of different vaccines.

### DETAILED DESCRIPTION

The technical solutions of the present disclosure will be clearly and completely described below with reference to the drawings of the present disclosure, and apparently, the described examples are only a part of the examples of the present disclosure, rather than all of them. Based on the examples of the present disclosure, all other examples obtained by those of ordinary skill in the art without creative efforts shall fall within the protection scope of the present disclosure.

### Example 1: Preparation of mRNA-LNP Complex

Taking SARS-CoV2 virus S protein antigen mRNA as a model nucleic acid, such LNP-mRNA vaccines were constructed as follows: ALC-0315/SM-102, DSPC, cholesterol, and ALC-0159/DMG-PEG2000 were dissolved in ethanol in a molar ratio of 47:10:41.5:1.5, mRNA was dissolved in an acetic acid buffer at pH 4.0, and an mRNA-LNP composition was prepared using a microfluidic device (nanoparticle preparation apparatus Ignite from Precision Nanosystems) with a nitrogen-to-phosphorus ratio of 6:1 according to a lipid mixture:mRNA flow rate ratio of 1:3. The packaged mRNA-LNP was dialyzed and concentrated by ultrafiltration into a phosphate buffer or a Tris buffer, and sterilely filtered to obtain a sample for a subsequent animal experiment. The settings for experimental sample groups are shown in Table 1. The samples were taken for the determination of encapsulation efficiency, average particle size, PDI, and Zeta potential.

**Table 1. Design for experimental groups**

| No. | Cationic lipid | PEG-lipid | Phospholipid | Steroid | Buffer |
|---|---|---|---|---|---|
| a | ALC-0315 | DMG-PEG2000 | DSPC | Cholesterol | Phosphate buffer |
| b | ALC-0315 | DMG-PEG2000 | DSPC | Cholesterol | Tris buffer |
| c | ALC-0315 | ALC-0159 | DSPC | Cholesterol | Phosphate buffer |
| d | ALC-0315 | ALC-0159 | DSPC | Cholesterol | Tris buffer |
| e | SM-102 | DMG-PEG2000 | DSPC | Cholesterol | Phosphate buffer |
| f | SM-102 | DMG-PEG2000 | DSPC | Cholesterol | Tris buffer |
| g | SM-102 | ALC-0159 | DSPC | Cholesterol | Phosphate buffer |
| h | SM-102 | ALC-0159 | DSPC | Cholesterol | Tris buffer |

**Table 2. Determination of encapsulation efficiency, average particle size, PDI, and Zeta potential**

| Group | Encapsulation efficiency (%) | Average particle size (nm) | PDI | Zeta potential (mV) |
|---|---|---|---|---|
| a | 93 | 70 | 0.052 | -6 |
| b | 98 | 68 | 0.038 | -7 |
| c | 92 | 60 | 0.057 | -5 |
| d | 93 | 68 | 0.052 | -3 |
| e | 90 | 78 | 0.081 | -7 |
| f | 91 | 68 | 0.062 | -2 |
| g | 89 | 72 | 0.121 | -5 |
| h | 92 | 67 | 0.083 | -6 |

As shown in Table 2, the results show that the experimental groups a, b, c, d, and h had encapsulation efficiency of ≥ 92%, among which group b had the highest encapsulation efficiency; all groups had comparable particle sizes; groups a, b, c, d, and f had relatively low PDI, among which group b had the lowest PDI; groups a, b, e, and h had relatively high Zeta potential. The encapsulation efficiency is a key attribute of LNP-mRNA, representing the proportion of mRNA encapsulated in the lipid nanoparticles out of the total mRNA. The mRNA encapsulated in the lipid nanoparticles can be protected from enzymatic degradation during delivery and effectively delivered into cells. The encapsulation efficiency of the lipid nanoparticles directly affects the stability and efficacy of the nanoparticles in their application. Monodispersity (PDI) represents the uniformity of the size distribution of particles, and a lower PDI value indicates that the lipid nanoparticles are more uniform in size, which ensures the stability of the product in clinical applications. Maintaining a slightly negative surface potential can increase the electrical repulsion force among LNPs, thereby maintaining the stability of individual particles, and it can also prevent the binding to oppositely charged proteins or other substances *in vivo* caused by the overly high absolute value of the potential, thereby improving the safety and bioavailability of the nanoparticles *in vivo.* Generally, it is preferable to maintain a slightly negative surface potential within -10 mV. Therefore, the experimental results in Table 2 show that the formulation of group b had a higher encapsulation efficiency, a smaller PDI, and a surface potential that meets the basic requirements, indicating that the formulation of group b has better application effects and performance.

### Example 2: Stability Study

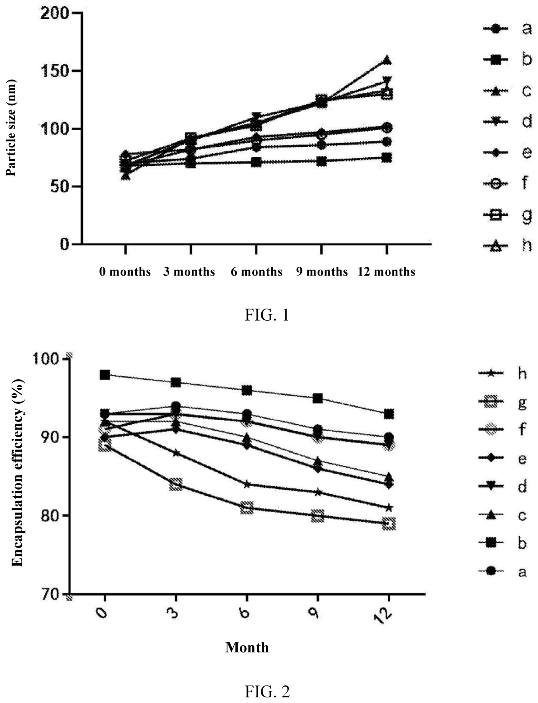

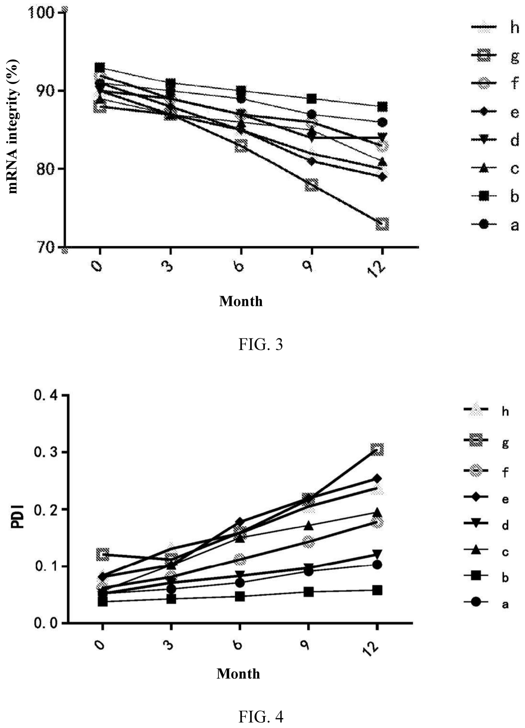

The mRNA-LNP complexes of groups a-h in Example 1 were each stored in a refrigerator at - 20 °C, and samples were taken every 3 months for the determination of encapsulation efficiency, average particle size, PDI, and mRNA integrity. The results are shown in FIG. 1, FIG. 2, FIG. 3, FIG. 4, Table 3, Table 4, Table 5, and Table 6 below.

**Table 3. Storage stability - average particle size**

| Group | Average particle size (nm) | | | | |
|---|---|---|---|---|---|
| | 0 months | 3 months | 6 months | 9 months | 12 months |
| a | 70 | 74 | 84 | 86 | 89 |
| b | 68 | 70 | 71 | 72 | 75 |
| c | 60 | 92 | 105 | 122 | 160 |
| d | 68 | 90 | 110 | 123 | 141 |
| e | 78 | 82 | 93 | 97 | 102 |
| f | 68 | 82 | 90 | 95 | 101 |
| g | 72 | 92 | 103 | 125 | 130 |
| h | 67 | 91 | 105 | 124 | 133 |

**Table 4. Storage stability - encapsulation efficiency (%)**

| Group | Encapsulation efficiency (%) | | | | |
|---|---|---|---|---|---|
| | 0 months | 3 months | 6 months | 9 months | 12 months |
| a | 93 | 94 | 93 | 91 | 90 |
| b | 98 | 97 | 96 | 95 | 93 |
| c | 92 | 92 | 90 | 87 | 85 |
| d | 93 | 93 | 92 | 90 | 89 |
| e | 90 | 91 | 89 | 86 | 84 |
| f | 91 | 93 | 92 | 90 | 89 |
| g | 89 | 84 | 81 | 80 | 79 |
| h | 92 | 88 | 84 | 83 | 81 |

**Table 5. Storage stability - mRNA integrity (%)**

| Group | mRNA integrity (%) | | | | |
|---|---|---|---|---|---|
| | 0 months | 3 months | 6 months | 9 months | 12 months |
| a | 91 | 90 | 89 | 87 | 86 |
| b | 93 | 91 | 90 | 89 | 88 |
| c | 89 | 87 | 86 | 85 | 81 |
| d | 90 | 89 | 87 | 84 | 84 |
| e | 91 | 88 | 85 | 81 | 79 |
| f | 92 | 89 | 87 | 86 | 83 |
| g | 88 | 87 | 83 | 78 | 73 |
| h | 90 | 87 | 85 | 82 | 80 |

**Table 6. Storage stability - PDI change**

| Group | PDI | | | | |
|---|---|---|---|---|---|
| | 0 months | 3 months | 6 months | 9 months | 12 months |
| a | 0.052 | 0.06 | 0.071 | 0.091 | 0.103 |
| b | 0.038 | 0.043 | 0.047 | 0.055 | 0.058 |
| c | 0.057 | 0.102 | 0.15 | 0.172 | 0.195 |
| d | 0.052 | 0.071 | 0.083 | 0.097 | 0.121 |
| e | 0.081 | 0.102 | 0.178 | 0.219 | 0.254 |
| f | 0.062 | 0.081 | 0.112 | 0.143 | 0.178 |
| g | 0.121 | 0.112 | 0.159 | 0.217 | 0.305 |
| h | 0.083 | 0.131 | 0.158 | 0.205 | 0.237 |

The results show that the samples of group b showed no significant decrease in the encapsulation efficiency and mRNA integrity within 12 months, showed no significant increase in the average particle size and PDI within 12 months, and exhibited better stability than the samples of the other groups.

### Example 3: Safety Study

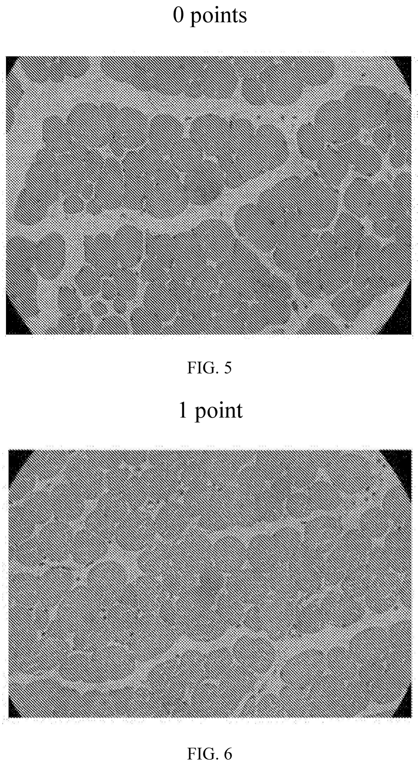

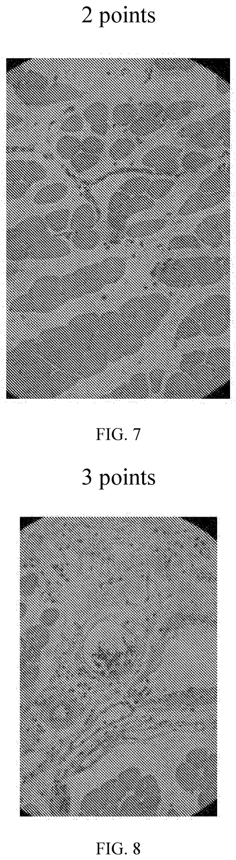

Female CD1 mice aged 6-8 weeks were randomly divided into 8 groups with 6 mice per group, and immunized via intramuscular injection in the hind leg. The single immunization dose of mRNA-LNP was 30 µg. The mice were tested for cytokine indexes such as IL-6 before the administration and 5 h after the administration (5H), and on day 3 (D3), and were sacrificed on D3. The muscle tissue at the injection site was fixed and routinely stained for the determination of inflammation, which was then scored on a 4-grade (0-3 points) method to evaluate local tissue tolerance.
Grade 1, referring to FIG. 5: no inflammation = 0 points;
Grade 2, referring to FIG. 6: mild inflammation = 1 point;
Grade 3, referring to FIG. 7: moderate inflammation = 2 points;
Grade 4, referring to FIG. 8, severe inflammation (more than 50% inflammatory cell infiltration) = 3 points.

**Table 7. Scoring of an inflammatory response**

| Group | Scoring of an inflammatory response | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| a | 1 | 2 | 2 | 1 | 1 | 1 | 1 | 3 | 3 | 3 | 1 | 1 |
| b | 1 | 1 | 0 | 1 | 1 | 1 | 2 | 0 | 1 | 1 | 2 | 2 |
| c | 1 | 1 | 2 | 0 | 3 | 1 | 1 | 2 | 3 | 3 | 1 | 1 |
| d | 2 | 2 | 1 | 1 | 1 | 1 | 2 | 1 | 1 | 1 | 3 | 2 |
| e | 2 | 1 | 2 | 3 | 3 | 3 | 2 | 1 | 1 | 3 | 1 | 1 |
| f | 1 | 1 | 1 | 1 | 1 | 1 | 2 | 2 | 1 | 1 | 3 | 1 |
| g | 1 | 1 | 1 | 2 | 3 | 1 | 1 | 3 | 1 | 1 | 2 | 3 |
| h | 2 | 2 | 1 | 1 | 1 | 1 | 1 | 1 | 3 | 3 | 1 | 1 |

**Table 8. Assay values of cytokines**

| Group | 5H | | D3 | |
|---|---|---|---|---|
| | IL-6 | KC/GRO | IL-6 | KC/GRO |
| a | 5293 | 1421 | 682 | 203 |
| b | 3197 | 1007 | 578 | 166 |
| c | 13243 | 1871 | 739 | 269 |
| d | 8928 | 1561 | 782 | 233 |
| e | 14232 | 1790 | 893 | 250 |
| f | 10087 | 1221 | 982 | 304 |
| g | 8237 | 1621 | 609 | 289 |
| h | 7159 | 1487 | 590 | 263 |

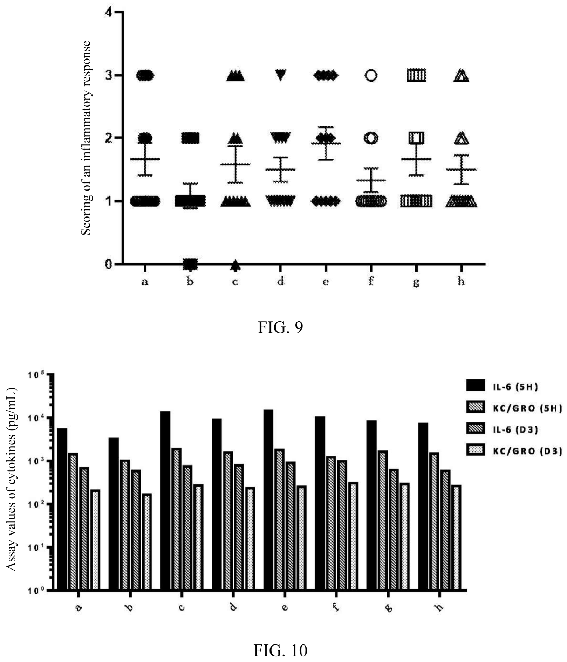

The results are shown in FIG. 9, FIG. 10, Table 7, and Table 8. The results show that the lipid nanoparticles of the samples of group b caused the mildest local inflammatory response, led to lower levels of inflammation-related cytokines, and had a lower risk of cytokine storm, thereby exhibiting better safety.

### Example 4: Biological Activity Study

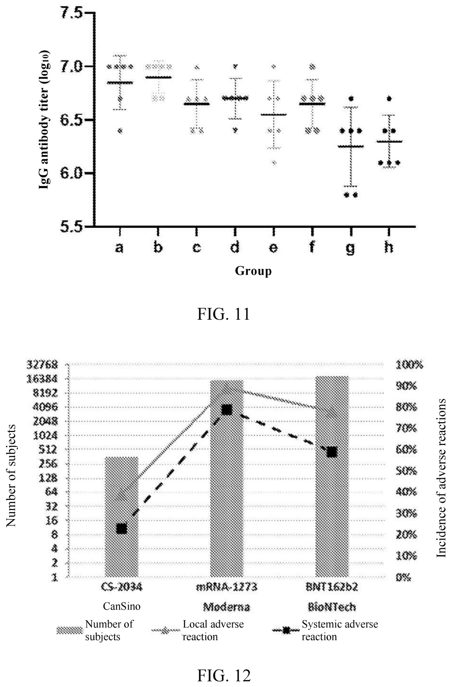

Female BALB/c mice aged 6-8 weeks were randomly divided into 8 groups with 6 mice per group, and immunized via intramuscular injection in the hind leg. Immunization was performed on day 0 and day 14, with an immunization dose of 5 µg mRNA-LNP. Blood was collected and serum was isolated on day 28 of immunization, and the specific antibody titer for the SARS-CoV2 virus S protein antigen was determined by the ELISA method (starting at a dilution factor of 80,000, two-fold gradient dilution). The results are shown in Table 9 and FIG. 11.

**Table 9. Assay values of antibody titer after immunization of mice (log10)**

| Group | Assay values of antibody titer | | | | | |
|---|---|---|---|---|---|---|
| | Mouse 1 | Mouse 2 | Mouse 3 | Mouse 4 | Mouse 5 | Mouse 6 |
| A | 7 | 6.7 | 6.4 | 7 | 7 | 7 |
| B | 7 | 6.7 | 7 | 7 | 7 | 6.7 |
| C | 6.7 | 6.4 | 6.7 | 7 | 6.7 | 7 |
| D | 6.7 | 6.7 | 6.4 | 6.7 | 7 | 6.7 |
| E | 6.7 | 6.7 | 6.7 | 6.1 | 6.1 | 6.4 |
| F | 6.7 | 6.7 | 6.4 | 6.4 | 7 | 6.7 |
| G | 5.8 | 6.1 | 6.1 | 5.8 | 6.4 | 6.1 |
| H | 6.1 | 5.8 | 5.2 | 6.1 | 6.1 | 5.2 |

The results show that the mRNA vaccine compositions formed by lipid nanoparticles of the samples of group b had higher antibody titer levels.

### Example 5: Stability of LNPs in Dilution Buffers at Different pH

Following the procedures in Example 1, mRNA-LNPs were prepared according to the formula of group b in Table 1, and the LNPs were added to Tris buffers at pH 5.0, 5.5, 6.0, 6.5, 7.0, 7.5, 8.0, 8.5, and 9.0 for dilution, respectively, to investigate the long-term stability after storage at -20 °C for 1 month.

**Table 10. Changes in the stability of mRNA-LNPs in buffers at different pH**

| Buffer pH | Evaluation index | Initial value | Storage at -20 °C for 1 month |
|---|---|---|---|
| pH 5.0 | Particle size (nm) | 55 | 65 |
| | PDI | 0.101 | 0.143 |
| | Encapsulation efficiency (%) | 99 | 98 |
| | Integrity (%) | 90 | 80 |
| pH 5.5 | Particle size (nm) | 61 | 70 |
| | PDI | 0.082 | 0.101 |
| | Encapsulation efficiency (%) | 99 | 98 |
| | Integrity (%) | 90 | 83 |
| pH 6.0 | Particle size (nm) | 65 | 70 |
| | PDI | 0.066 | 0.078 |
| | Encapsulation efficiency (%) | 99 | 95 |
| | Integrity (%) | 92 | 90 |
| pH 6.5 | Particle size (nm) | 71 | 75 |
| | PDI | 0.041 | 0.052 |
| | Encapsulation efficiency (%) | 97 | 95 |
| | Integrity (%) | 92 | 90 |
| pH 7.0 | Particle size (nm) | 68 | 69 |
| | PDI | 0.036 | 0.035 |
| | Encapsulation efficiency (%) | 98 | 97 |
| | Integrity (%) | 92 | 92 |
| pH 7.5 | Particle size (nm) | 69 | 70 |
| | PDI | 0.034 | 0.032 |
| | Encapsulation efficiency (%) | 98 | 98 |
| | Integrity (%) | 93 | 92 |
| pH 8.0 | Particle size (nm) | 68 | 72 |
| | PDI | 0.031 | 0.041 |
| | Encapsulation efficiency (%) | 96 | 96 |
| | Integrity (%) | 93 | 91 |
| pH 8.5 | Particle size (nm) | 71 | 74 |
| | PDI | 0.042 | 0.051 |
| | Encapsulation efficiency (%) | 96 | 95 |
| | Integrity (%) | 93 | 90 |
| pH 9.0 | Particle size (nm) | 75 | 81 |
| | PDI | 0.064 | 0.091 |
| | Encapsulation efficiency (%) | 95 | 90 |
| | Integrity (%) | 91 | 80 |

According to the above experimental results, as shown in Table 10, the mRNA integrity was slightly decreased at low pH (pH 5.0) and high pH (pH 9.0). The pH level affected the mRNA integrity, which in turn affected the stability of the mRNA-LNP formulations. The present disclosure achieved an optimized pH range of 6.0 to 8.5 through experimental optimization, and there were relatively small differences in the encapsulation efficiency, average particle size, PDI, and mRNA integrity of mRNA-LNPs. This suggests that the mRNA-LNPs have better stability in the range of buffer pH (pH 6.0-8.5) provided by the present disclosure.

### Example 6: Potency Assay for LNPs with Different Lipid Formulas

Following the procedures in Example 1, LNP formulations with different formulas were prepared for the preparation of mRNA-LNPs, and were investigated for immunogenicity in mice.

**Table 11. Comparison results for LNPs prepared according to different lipid formulas**

| Experiment No. | ALC-0315:DSPC:cholesterol:DMG -PEG2000 (molar ratio) | Encapsulation efficiency (%) | Average particle size (nm) | Serum IgG antibody titer (Log value), GMT (95% CI) |
|---|---|---|---|---|
| 1 | 80:1:10:9 | 92 | 62 | 6.7 (6.5-6.8) |
| 2 | 74:1:10:15 | 94 | 58 | 6.7 (6.5-6.8) |
| 3 | 70:2:25:3 | 90 | 69 | 6.7 (6.5-6.8) |
| 4 | 60:2:35:3 | 93 | 70 | 6.7 (6.5-6.8) |
| 5 | 60:10:17:13 | 90 | 61 | 6.7 (6.5-6.8) |
| 6 | 60:20:19.9:0.1 | 91 | 81 | 6.7 (6.5-6.8) |
| 7 | 60:5:30:5 | 93 | 67 | 6.7 (6.5-6.8) |
| 8 | 54.5:15:30:0.5 | 92 | 78 | 6.7 (6.5-6.8) |
| 9 | 52:10:36.5:1.5 | 96 | 75 | 6.9 (6.7-7) |
| 10 | 49:10:39.5:1.5 | 97 | 72 | 6.9 (6.7-7) |
| 11 | 47:10:41.5:1.5 | 98 | 70 | 6.9 (6.7-7) |
| 12 | 45:10:43.5:1.5 | 97 | 71 | 6.9 (6.7-7) |
| 13 | 44:10:44.5:1.5 | 98 | 69 | 6.9 (6.7-7) |
| 14 | 43.5:10:45:1.5 | 96 | 71 | 6.9 (6.7-7) |
| 15 | 41:10:47.5:1.5 | 97 | 75 | 6.9 (6.7-7) |
| 16 | 38:10:50.5:1.5 | 97 | 78 | 6.9 (6.7-7) |
| 17 | 35:10:53.5:1.5 | 92 | 70 | 6.7 (6.5-6.8) |
| 18 | 30:5:60:5 | 93 | 65 | 6.7 (6.5-6.8) |
| 19 | 30:20:40:10 | 91 | 62 | 6.7 (6.5-6.8) |

According to the above experimental results, as shown in Table 11, when the cationic lipid content in the formulas provided by the present disclosure was 30%-80%, the LNPs all had the encapsulation efficiency of no less than 90% and the antibody titer of about 6.7, and exhibited relatively good encapsulation efficiency and immunogenicity; when the cationic lipid content of the formulas provided was 38%-52, the LNPs all had the encapsulation efficiency of no less than 96% and the antibody titer of about 6.9, and no significant difference existed. This suggests that the lipid formulas provided by the present disclosure have better encapsulation efficiency and immunogenicity by adjusting the content of cationic lipids in the liposomes.

### Example 7: Clinical Trial Results for mRNA-LNP Vaccine

With reference to the preparation method in Example 1, the SARS-CoV-2 COVID-19 mRNA-LNP vaccine was prepared according to the LNP formula (ALC-0315:DSPC:cholesterol:DMG-PEG2000 = 47:10:41.5:1.5) of the present disclosure and using a Tris dilution buffer at pH 7.5. Moreover, the efficacy and safety of the vaccine in humans were investigated in a clinical trial. The results are shown in Table 11.

**Table 12. Statistics of side effects of different vaccines in clinical trials**

| Vaccine group | CanSino CS-2034 | Moderna¹ mRNA-1273 | BioNTech² BNT162b2 |
|---|---|---|---|
| Number of subjects | 353 | 15181 | 18860 |
| Local adverse reaction | 39% | 89% | 78% |
| Systemic adverse reaction | 23% | 79% | 59% |

| | | | |
|---|---|---|---|
| Note 1: Data are cited from the literature article Baden, L.R., et al., Efficacy and Safety of the mRNA-1273 SARS-CoV-2 Vaccine. N Engl J Med, 2021. 384 (5): p. 403-416. Note 2: Data are cited from the literature article Polack, F.P., et al., Safety and Efficacy of the BNT162b2 mRNA Covid-19 Vaccine. N Engl J Med, 2020. 383 (27): p. 2603-2615. | | | |

In the phase II clinical trial, 353 adults were vaccinated, including 120 subjects aged 18-59 and 273 subjects aged over 60. After administration by intramuscular injection of 30 µg of the vaccine drug, the occurrence of adverse reactions within 28 days of vaccination was counted. The local adverse reactions include pain, swelling, erythema, induration, etc., at the injection site, and the systemic adverse reactions include fever, headache, fatigue, joint pain, muscle pain, nausea, diarrhea, vomiting, cough, etc. The statistical results are shown in Table 11 and FIG. 12. Comparing to the public data for the two marketed vaccines, i.e., mRNA-1273 and BNT162b2, the vaccine provided by the present disclosure had better safety, with much lower incidences of local and systemic adverse reactions than mRNA-1273 and BNT162b2. This suggests that the lipid nanoparticles provided by the present disclosure have better safety.

The preferred embodiments of the present disclosure are described in detail above, which, however, are not intended to limit the present disclosure. Within the scope of the technical concept of the present disclosure, various simple modifications can be made to the technical solution of the present disclosure, all of which will fall within the protection scope of the present disclosure.

In addition, it should be noted that the various specific technical features described in the above specific embodiments can be combined in any suitable manner in which the features do not contradict each other. In order to avoid unnecessary repetition, such combinations will not be illustrated separately.

## Claims

1. A lipid nanoparticle composition for nucleic acid drug delivery, comprising the following starting materials:
(a) an ionizable cationic lipid, wherein preferably, the ionizable cationic lipid accounts for 30%-80% of total lipid present in the lipid nanoparticle on the basis of molar percentage;
(b) a helper phospholipid, wherein preferably, the helper phospholipid is DSPC, and more preferably, the helper phospholipid accounts for 1%-20% of total lipid present in the lipid nanoparticle on the basis of molar percentage;
(c) a sterol compound, wherein preferably, the sterol compound is cholesterol, and more preferably, the sterol compound accounts for 10%-60% of total lipid present in the lipid nanoparticle on the basis of molar percentage;
(d) a lipid-polyethylene glycol conjugate, wherein preferably, the lipid-polyethylene glycol conjugate is one or more of DMG-PEG2000 and ALC-0159, and more preferably, the lipid-polyethylene glycol conjugate accounts for 0.1%-15% of total lipid present in the lipid nanoparticle on the basis of molar percentage;
(e) a buffer, wherein preferably, the buffer is selected from one or more of a tris buffer, an acetate buffer, a citrate buffer, and a phosphate buffer.

2. The lipid nanoparticle composition according to claim 1, wherein the ionizable cationic lipid is one or more of ALC-0315 and SM-102.

3. The lipid nanoparticle composition according to any one of claims 1-2, wherein the buffer has a pH of 5-9, preferably a pH of 6-8.5.

4. The lipid nanoparticle composition according to any one of claims 1-3, wherein the ALC-0315 accounts for 38%-52% of total lipid present in the lipid nanoparticle on the basis of molar percentage.

5. The lipid nanoparticle composition according to any one of claims 1-4, wherein the buffer is at a concentration of 1-100 Mm, preferably a concentration of 5-50 mM.

6. The lipid nanoparticle composition according to any one of claims 1-5, further comprising an additional auxiliary material, wherein the additional auxiliary material comprises one of sodium acetate, acetic acid, tromethamine, potassium dihydrogen phosphate, sodium chloride, disodium hydrogen phosphate, and sucrose or combinations thereof.

7. The lipid nanoparticle composition according to any one of claims 1-6, further comprising a nucleic acid drug, wherein the nucleic acid drug comprises ssDNA, dsDNA, mRNA, lncRNA, siRNA, saRNA, shRNA, ASO, a plasmid, circRNA, circDNA, miRNA, CRISPR-Cas, ployI:C, SamRNA, or 5'-pppRNA; preferably, the nucleic acid drug is mRNA.

8. The lipid nanoparticle composition according to any one of claims 1-7, wherein the composition is a liquid formulation or a lyophilized powder formulation; preferably, the composition is a formulation for oral administration, intramuscular injection, intravenous injection, subcutaneous injection, or inhalation.

9. A preparation method for the nucleic acid-lipid nanoparticle composition according to any one of claims 1-8, wherein the composition is prepared by dissolving the ionizable cationic lipid, the helper phospholipid, the sterol compound, and the PEG-lipid conjugate in a solvent, mixing the solution with a nucleic acid drug, diluting the mixture with the buffer, and concentrating the dilution.

10. The preparation method for the lipid nanoparticle composition according to claim 9, wherein the mass ratio (w/w) of the total lipid in the lipid nanoparticle (LNP) to mRNA is (10-30):1.

11. The preparation method for the lipid nanoparticle composition according to any one of claims 9-10, wherein the lipid nanoparticle has an average particle size of 50-200 nm; or the lipid nanoparticle has a net neutral charge at neutral pH; or the lipid nanoparticle has a polydispersity value of less than 0.4.

12. The preparation method for the lipid nanoparticle composition according to any one of claims 9-11, wherein the lipid nanoparticle has a N/P ratio of 1-10 when encapsulating mRNA.

13. Use of the lipid nanoparticle composition according to any one of claims 1-12 in the manufacture of a prophylactic or therapeutic medicament, wherein preferably, the medicament is a vaccine, and more preferably, the vaccine is a vaccine for preventing a cancer, a viral infection, a bacterial infection, or a fungal infection; more preferably, the virus is selected from norovirus, Ebola virus, coronavirus, cytomegalovirus, dengue virus, Zika virus, coxsackievirus, enterovirus, hepatitis virus, herpes simplex virus, human papilloma virus, influenza virus, Marburg virus, measles virus, poliovirus, rabies virus, rotavirus, and measles virus.
